# FASCICULE DE BREVET EUROPEEN

(11) **EP 1 890 610 B1**
(45) Date de publication et mention de la délivrance du brevet: **24.09.2008**
(21) Numéro de dépôt: 06709394.8
(22) Date de dépôt: 09.01.2006
(51) Int. Cl.: A61B 17/04, A61B 17/06, A61B 17/32, A61B 17/00

(54) **AIGUILLE CHIRURGICALE ET FIL UTILISE AVEC CETTE AIGUILLE**
CHIRURGISCHE NADEL UND DAMIT VERWENDETES NAHTMATERIAL
SURGICAL NEEDLE AND SUTURE USED THEREWITH

(30) Priorité: 07.01.2005 FR 0550069
(43) Date de publication de la demande: 27.02.2008
(73) Titulaire: Cumbo, Peter, 95590 Presles (FR)
(72) Inventeur: Cumbo, Peter, 95590 Presles (FR)
(74) Mandataire: Lebrette, Camille
(86) Numéro de dépôt international: PCT/FR2006/050006
(87) Numéro de publication internationale: WO 2006/072754

(56) Documents cités:
- WO-A-20/04002326
- US-A- 5 507 755

## Description

La présente invention concerne une aiguille chirurgicale et un fil utilisé avec cette aiguille. L'invention a notamment pour but de permettre le déplacement d'une telle aiguille sous la peau d'un patient selon une trajectoire déterminée par l'opérateur, par exemple en boucle. L'invention trouve une application particulièrement avantageuse dans le domaine de la chirurgie plastique, mais elle pourrait aussi être utilisée dans d'autres domaines de la chirurgie.

La demande de procédés sûrs, tout en étant «légers» c'est à dire à faible morbidité et sans éviction sociale prolongée est de plus en plus forte dans le domaine de la chirurgie esthétique notamment.

Il existe des techniques utilisant des fils sous-cutanés visant à remettre en tension les tissus (du visage ou du corps) d'un sujet sans cicatrice apparente ou du moins avec une rançon cicatricielle minime. Néanmoins, ces techniques ne sont pas toujours aisées à mettre en oeuvre et leurs résultats ne sont pas constants.

Le document WO2004/002326, décrit une aiguille chirurgicale qui comporte un trou permettant l'accrochage du fil à l'aiguille. Toutefois WO2004/002326 ne décrit, ni ne suggère que le trou est "allongé", qu'il s'étend "dans le sens de l'allongement de l'aiguille", et qu'il présente "une longueur supérieure a 30% de la longueur de l'aiguille" pour permettre le coulissement du à l'intérieur de l'aiguille.

En effet, il s'agit généralement d'utiliser des fils spécialement conçus pour «accrocher» les tissus grâce à leur «crantage». Ceux-ci sont introduits sous la peau à l'aide de cathéters creux rectilignes qui ne permettent que des trajectoires simples; une trajectoire «aller et retour» est très difficile à réaliser.

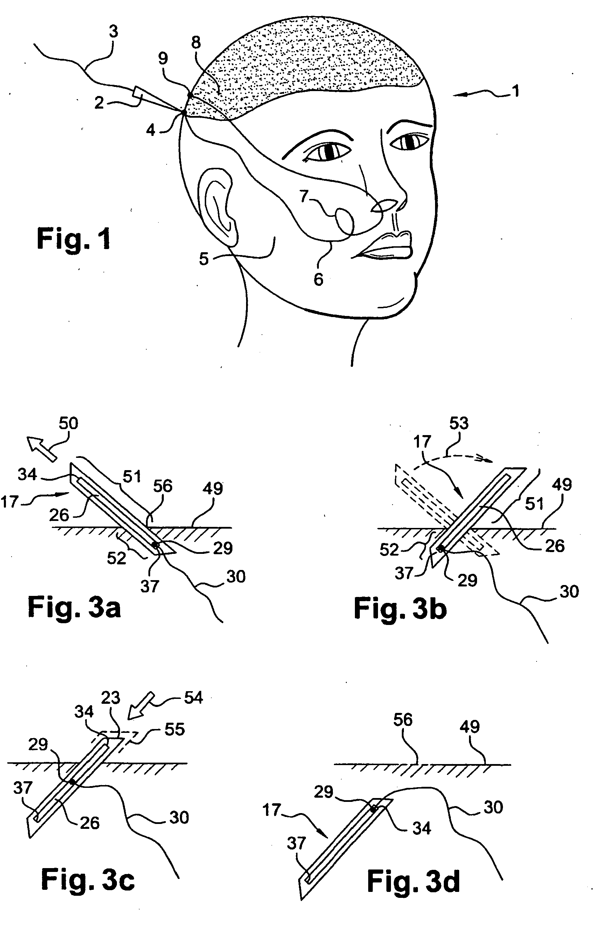

La figure 1 montre une représentation schématique du visage d'un patient 1 dont des tissus de la peau du visage vont être retendus. L'opération s'apparente à une sorte de lifting sans intervention chirurgicale vraie, sans cicatrice visible. Elle se pratique sous anesthésie locale pure sans aucune hospitalisation et est rapide à mettre en oeuvre.

En pratique, dans cet exemple, afin de retendre les tissus de la pommette 5, une aiguille 2 entraînant un fil 3 est introduite sous la peau par une incision 4 dans la région temporale du patient 1. Cette aiguille 2 décrit sous la peau une trajectoire 6 pré-établie qui a pu être préalablement dessinée sur le visage du patient 1. Cette trajectoire 6 est sinueuse, comportant si nécessaire une ou plusieurs boucles 7 ou autre figure en fonction du résultat recherché, à savoir notamment en fonction de la zone de tissu à retendre. La trajectoire 6 de l'aiguille 2 est en général déterminée de manière à ce que l'aiguille 2 ressorte dans l'incision de départ 4, ou dans une zone 9 proche de ce point.

Une fois que le fil 3 est ressorti, les deux brins sont mis en tension afin d'obtenir l'effet souhaité et sont noués en prenant éventuellement appui sur une structure solide telle que l'aponévrose temporale bien connue des chirurgiens plasticiens. La cicatrice résiduelle, minime, est située dans une zone chevelue et donc dissimulée. Dans certains cas, une résection minime de peau peut être effectuée dans cette zone.

Les procédés actuels font appel à un cathéter cylindrique creux et rectiligne comportant une pointe à une extrémité et un orifice simple de l'autre et qui sert de guide pour l'introduction du fil, en général cranté. Le cathéter est introduit sous la peau seul en suivant la trajectoire décidée, puis la pointe est extériorisée au point terminal de la dite trajectoire et le fil passé de façon rétrograde (par la pointe) dans le corps du cathéter pour ressortir au point d'entrée de celui-ci. Le cathéter est alors retiré et le fil est en place sous la peau. Il ne reste plus qu'à le mettre en tension après avoir «ramassé» les tissus sur les crans du fil. Il est aisé d'imaginer les limites d'une telle technique.

De tels cathéters sont susceptibles de décrire des trajectoires rectilignes, voire des trajectoires courbes ou légèrement sinueuses. Toutefois, ils ne permettent pas de décrire notamment une boucle complète afin de faire ressortir l'extrémité du fil en un endroit proche de son point d'introduction. Il est difficile dans ces conditions d'effectuer une véritable remise en tension des tissus avec fixation pour assurer un bon maintien.

En effet, si le praticien souhaitait faire suivre au cathéter une telle trajectoire, il serait obligé de décomposer cette dernière en plusieurs étapes en faisant ressortir le fil à chaque fois tout en sachant qu'il est tout à fait improbable de réussir à réintroduire le fil par le même orifice.

L'invention ci-après décrite propose une solution aux problèmes évoqués précédemment en permettant de faire progresser un fil au sein des tissus du sujet selon n'importe quelle trajectoire sans jamais avoir à l'extérioriser avant d'atteindre sa destination finale.

A cette fin, l'aiguille faisant l'objet de l'invention est creuse, comporte deux extrémités, chacune se terminant par une pointe, ainsi qu'un orifice pour introduire le fil et une fente longitudinale. L'orifice et la fente sont situés sur les parois opposées du corps creux de l'aiguille. L'extrémité du fil utilisé est nouée ou sertie d'une sorte de «plomb» dont le diamètre est inférieur à celui de l'orifice de l'aiguille de telle manière à permettre son passage, mais de diamètre supérieur à la largeur de la fente qui par conséquent retiendra le fil et permettra son entraînement par l'aiguille.

Ainsi le fil est introduit dans l'orifice et, à travers l'orifice, dans la fente par son extrémité libre (non sertie). Puis, on tire sur l'extrémité libre jusqu'à ce que le noeud ou le plomb pénètre dans l'orifice et soit bloquée par la fente.

De cette manière, le fil est libre de coulisser le long de la fente de l'aiguille tout en restant solidaire de celle-ci, et pourra suivre les trajectoires déterminées par l'opérateur «à la demande».

En effet, il est possible, au moment où un changement d'orientation est nécessaire, de sortir partiellement l'aiguille de la peau du sujet, en extériorisant l'une de ses pointes puis une partie de son corps, tout en maintenant l'autre pointe et le fil enfouis sous la peau. Puis, en faisant un petit mouvement de levier et en imprimant une rotation à l'aiguille, l'aiguille peut être redirigée dans la direction voulue et ré-enfouie totalement sous la peau et ainsi de suite en changeant à chaque fois de pointe directrice. Le fil coulisse le long de la fente et suit l'aiguille sans jamais s'extérioriser.

Comme l'aiguille est creuse et tranchante et qu'il n'est jamais nécessaire de ressortir le fil, même pour décrire les trajectoires les plus sinueuses, aucune cicatrice inesthétique ne sera laissée par l'aiguille. On observera tout au plus et très transitoirement les points de perforation cutanée laissés par l'aiguille comme lors de n'importe quelle injection sous-cutanée effectuée avec une aiguille ou un trocart. Il n'existe donc pas de véritable cicatrice, sauf à l'emplacement de l'incision de départ, généralement minime et dissimulée.

L'aiguille possède généralement une forme cylindrique rectiligne. Les pointes de l'aiguille sont biseautées et tranchantes afin de permettre une bonne pénétration dans les tissus.

Un fil comportant un arrêtoir (sorte de « plomb ») ou un renfort à une de ses extrémités peut être fourni et utilisé avec l'aiguille. Ce fil spécial présentera moins de risque de fragilisation dû au frottement lors du coulissement le long de la fente.

Le fil utilisé peut être résorbable ou non-résorbable, «cranté» ou non cranté au choix et selon les préférences du praticien.

L'invention concerne donc une aiguille chirurgicale comportant :
- un corps creux de forme allongée et deux extrémités; ce corps comportant une paroi ventrale et une paroi dorsale réunies entre elles, la paroi dorsale étant opposée géométriquement à la paroi ventrale, cette paroi dorsale et cette paroi ventrale étant allongées selon un allongement de l'aiguille, les deux extrémités creuses comportant chacune une pointe,
caractérisée en ce qu'elle comporte en outre :
- une fente ménagée dans la paroi ventrale, cette fente s'étendant entre les deux extrémités du corps.

L'invention concerne également un fil utilisé avec l'aiguille chirurgicale ci-dessus décrite et caractérisé en ce qu'il comporte un arrêtoir de dimensions intermédiaires entre le diamètre de l'orifice de l'aiguille et la plus grande largeur de la fente ventrale.

L'invention sera mieux comprise à la lecture de la description qui suit et à l'examen des figures qui l'accompagnent. Ces figures sont données à titre indicatif mais nullement limitatif de l'invention. Ces figures montrent :
- Figure 1 (déjà décrite) : une représentation schématique d'un visage de sujet dont on procède à la remise en tension des tissus ;
- Figure 2 : une représentation schématique d'une aiguille à deux pointes selon l'invention ;
- Figures 3 : une représentation schématique des différentes étapes d'une procédure d'utilisation de l'aiguille selon l'invention ;
- Figures 4 : des représentations schématiques de variantes de conception de l'aiguille selon l'invention ;
- Figures 5 : une représentation schématique d'un fil muni d'un arrêtoir et d'une variante possédant un renflement à une extrémité et conçus pour être utilisés avec l'aiguille selon l'invention,
- Figures 6 : des représentations schématiques précises d'une aiguille selon l'invention qui ne comporte pas d'orifice.

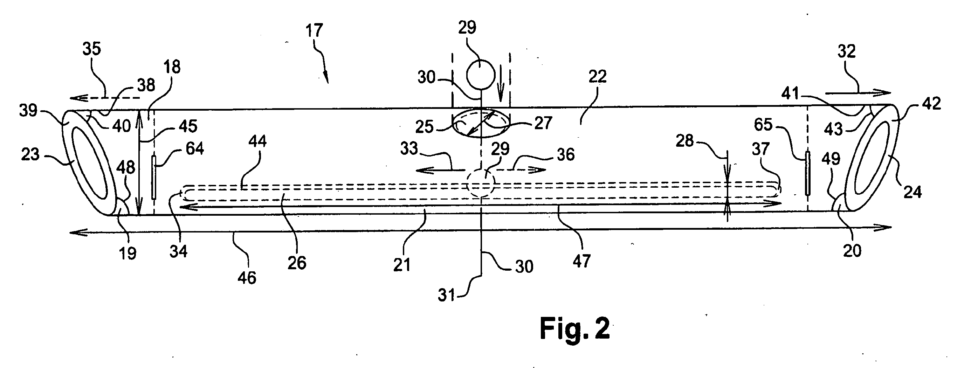

La figure 2 montre une aiguille chirurgicale 17 selon l'invention. Cette aiguille chirurgicale 17 comporte un corps 18 creux de forme allongée ou rectiligne et deux extrémités 19 et 20. Ce corps 18 comporte une paroi ventrale 21 et une paroi dorsale 22 réunies entre elles. La paroi dorsale 22 est opposée géométriquement à la paroi ventrale 21. Cette paroi dorsale 22 et cette paroi ventrale 21 sont allongées selon un allongement de l'aiguille 17. Comme l'aiguille 17 est ici cylindrique, cet allongement correspond à un axe de l'aiguille. Toutefois, en variante, l'aiguille 17 peut avoir une forme allongée autre qu'un cylindre, telle qu'une forme de croissant, dans laquelle l'allongement ne correspond pas à un axe.

Les deux extrémités 19 et 20 comportent chacune une pointe 23 et 24 creuse. Plus précisément, l'extrémité 19 se termine par une pointe 23 et l'extrémité 20 par une pointe 24. La terminaison de la pointe 23, 24 située à chaque extrémité de l'aiguille 17, constitue le plus souvent le prolongement de la paroi dorsale 22 de ladite aiguille 17. Toutefois, les pointes 23 et 24 ne sont pas nécessairement creuses vis-à-vis de l'invention. En effet, on pourrait réaliser une aiguille comportant des pointes 23 et 24 pleines.

L'aiguille 17 comporte en outre un orifice 25 ménagé dans la paroi dorsale 22 du corps 18, et une fente 26 ménagée dans la paroi ventrale 21. Cette fente 26 s'étend entre les deux extrémités 19 et 20 du corps 18 sans toutefois atteindre les-dites extrémités. Cette fente 26 est donc fermée de part et d'autre afin de toujours retenir un fil 30 chirurgical usuel qui coulisse en son sein. Dans une réalisation particulière, une longueur de cette fente 26 est supérieure à 10% de la longueur de l'aiguille et/ou supérieure à 5 fois le diamètre de l'aiguille.

Par ailleurs, une plus petite dimension de l'orifice 25 est supérieure à la plus grande largeur 28 de la fente 26. Comme l'orifice 25 est ici de forme circulaire et que la fente 26 est de forme rectangulaire, le diamètre 27 de l'orifice 25 est plus grand que la largeur de la fente 26. Dans une variante, la fente 26 est globalement de forme elliptique et le diamètre 27 de l'orifice 25 est plus grand que la plus grande largeur de cette fente 26 elliptique.

Les dimensions de l'orifice 25 et de la fente 26 sont ainsi déterminées de manière que l'orifice 25 autorise le passage d'un élément bloquant 29, tel qu'un noeud ou un épaississement (renflement) ou autre arrêtoir du fil 30, et que la fente 26 retienne cet élément bloquant 29. La fente 26 possède ainsi une largeur légèrement supérieure au diamètre du fil 30 utilisé, mais inférieure au diamètre du noeud ou arrêtoir 29 de ce fil 30. En outre, la longueur de la fente 26 est supérieure à la plus grande dimension de l'orifice.

Pour utiliser l'aiguille 17, on fait donc passer une extrémité 31 du fil 30 à l'intérieur de l'orifice 25 et de la fente 26. Une fois que le noeud 29 est positionné à l'intérieur de l'aiguille 17, il est susceptible de coulisser le long de la fente 26 tout en étant retenu à chaque extrémité de cette fente 26 qui est fermée. Ainsi, lorsque l'aiguille 17 se déplace suivant une direction rectiligne référencée 32 (extrémité 20 directrice), le noeud ou arrêtoir 29 coulisse le long de la fente 26 suivant une direction 33 opposée car le fil 30 ne sera entraîné par l'aiguille 17 qu'à partir du moment où il arrivera en butée contre l'extrémité 34 de la fente. Le fil 30 sera alors entraîné par l'aiguille 17 dont il épousera la trajectoire au sein des tissus.

A l'inverse, lorsque l'aiguille 17 se déplace suivant une direction 35 opposée à la direction 32 (extrémité 19 directrice), le noeud ou arrêtoir 29 se déplace le long de la fente 26 suivant une direction 36. Lorsque le noeud ou arrêtoir 29 arrive en butée contre l'extrémité 37 de la fente 26, il se bloque et se trouve ainsi entraîné avec le fil 30 dans le sillage de l'aiguille qui décrit la trajectoire voulue par l'opérateur au sein des tissus du sujet. A cette fin, les extrémités 34 et 37 sont généralement orientées perpendiculairement à l'allongement de l'aiguille 17.

Le corps 18 de l'aiguille 17 présente généralement une forme cylindrique mais des variantes sont possibles, telles qu'une forme tronconique ou la forme en croissant précitée, visant à optimiser notamment la pénétration de l'aiguille 17 ainsi que sa progression dans les tissus.

Chaque extrémité 34, 37 de la fente 26 est distante de l'extrémité 19, 20 la plus proche d'une longueur donnée, généralement de l'ordre de un à plusieurs millimètres, voire plus selon les dimensions de l'aiguille. La fente 26 a généralement une situation centrale et symétrique dans une des parois de l'aiguille 17, généralement la paroi 21 ventrale.

Les pointes 23,24 situées à chaque extrémité de l'aiguille 17 sont le plus souvent biseautées, de manière que la terminaison des pointes soit dans le prolongement de la paroi 22 dorsale de l'aiguille 17, c'est à dire que le biseau forme un angle aigu 40,43 avec la paroi dorsale 22 et un angle obtus 48,49 avec la paroi ventrale 21. La paroi dorsale 22 est par conséquent plus longue que la paroi ventrale 21.

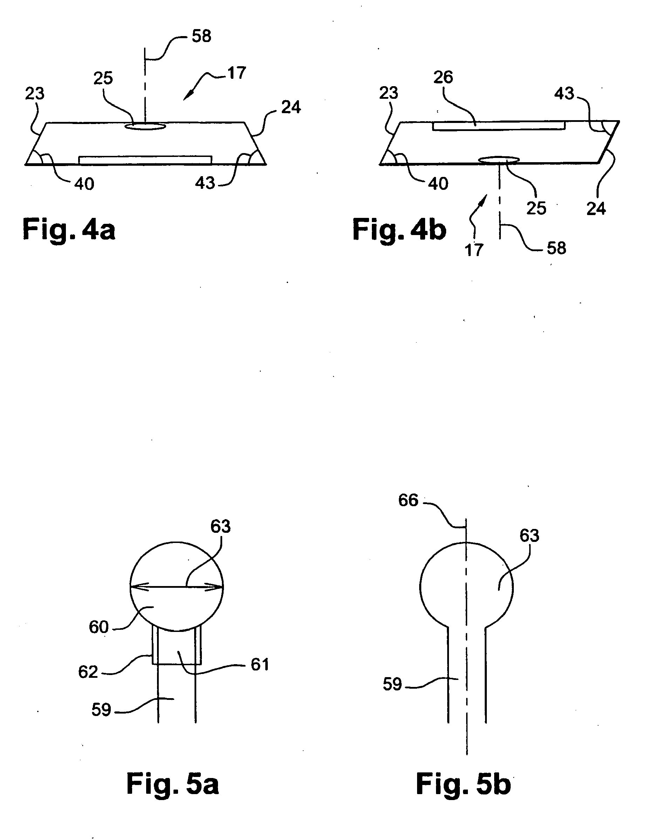

Toutefois, comme on le verra dans les figures 4, les biseaux des pointes 23,24 peuvent être orientés de différentes manières. En variante, les pointes 23,24 pourraient avoir une forme différente, telle qu'une forme conique.

Le corps 18 comporte un diamètre 45 de l'ordre du millimètre et une longueur 46 de plusieurs cm, comprise par exemple entre 5 et 20 cm. Le diamètre 27 de l'orifice 25 est dans un exemple de l'ordre du millimètre et est variable en fonction des dimensions de l'aiguille 17. La fente 26 peut s'étendre sur une longueur 47 comprise entre 4 et 15 cm. La fente 26 s'étend par exemple sur une longueur 47 inférieure de 5 à 15 mm à celle de la paroi dans laquelle elle se situe. Dans la pratique, les dimensions ci-dessus évoquées dépendent du type et du diamètre du fil 30 utilisé ainsi que de l'intervention projetée et de sa localisation (visage ou corps...).

L'aiguille 17 peut être fabriquée par exemple à partir d'une plaque d'acier d'une épaisseur et d'une flexibilité adaptées. Cette plaque serait alors enroulée et ses bords soudés de manière à obtenir une forme cylindrique. Plus précisément, cette plaque peut dans un premier temps être usinée de manière à créer l'orifice 25 ainsi que la fente 26 décrits précédemment puis enroulée. Les pointes biseautées seront réalisées par les techniques habituellement usitées pour la fabrication de cathéters et autres aiguilles et trocarts. Les pointes 23, 24 peuvent par exemple être soudées aux extrémités 19 et 20 du corps 18. Dans un autre exemple, l'aiguille 17 est obtenue par moulage. L'orifice 25 et la fente 26 pourraient aussi être confectionnés après création du corps de l'aiguille 18 et de ses deux pointes 23,24. L'aiguille 17 peut aussi être réalisée dans un autre matériau adapté à son utilisation.

Etant donné que le noeud 29 coulisse régulièrement le long de la fente 26, un chant 44 de la fente 26 situé dans l'épaisseur de la paroi de l'aiguille 17 peut être usiné, de manière à présenter une forme arrondie ou mousse et par conséquent non tranchante pour le fil. Dans un exemple, le chant 44 de la fente 26 est limé pour la rendre non tranchante. En variante, la fente 26 peut être recouverte d'un matériau non tranchant. Dans une autre variante, comme on le verra dans la figure 5, c'est le fil 30 qui est renforcé dans sa portion en contact avec les bords de la fente 26.

Un repère 64, 65 visuel tel qu'une encoche ou un trait, coloré ou non, peut être inséré proche de chaque extrémité de l'aiguille 17 afin d'en faciliter l'utilisation pour le praticien. Ce repère 64, 65 visuel permet au praticien de connaître globalement la longueur de l'aiguille 17 enfouie sous la peau du patient et d'éviter ainsi de faire ressortir le noeud 29 lors d'une opération. En effet, tant que le praticien ne voit pas le repère de la partie qui est enfouie, il peut tirer sur l'aiguille 17 sans faire sortir le noeud 29 d'un tissu de peau du patient. A cet effet, le repère 64, 65 s'étend généralement perpendiculairement à l'allongement de l'aiguille 17. Ce repère 64, 65 peut s'étendre dans un exemple sur toute une circonférence de l'aiguille 17, ou sur une partie de cette circonférence. Dans une réalisation, des repères visuels circulaires sont réalisés au laser à une distance des extrémités de 5mm environ, et possèdent une épaisseur de 0,4mm environ.

En variante, l'aiguille 17 ne comporte pas d'orifice 25. Dans cette variante, pour positionner le fil, on fait passer son extrémité libre par une des pointes creuses 23, 24, puis à travers l'aiguille 17, et par la fente 26. On tire ensuite sur cette extrémité libre jusqu'à ce que l'autre extrémité du fil, qui comporte un arrêtoir ou un noeud, entre en butée contre cette la fente 26. Un tel passage du fil à travers une pointe et la fente est rendu possible par le fait qu'une extrémité de la fente 26 est très proche de celle d'une pointe creuse, la distance les séparant étant de l'ordre de 1 à 3mm. Ainsi, lors des manipulations externes de l'aiguille, l'effet de levier permettant de faire tourner l'aiguille à l'intérieur des tissus est très favorable.

Les figures 3 montrent une représentation par étapes de l'utilisation de l'aiguille 17 selon l'invention.

La figure 3a montre une étape dans laquelle l'aiguille 17, après avoir effectué une portion de trajectoire sous-cutanée rectiligne ou légèrement courbe perfore la peau 49 du sujet en un point 56 prédéterminé où doit avoir lieu un changement de direction. Plus précisément, l'aiguille 17 est conduite à perforer la peau puis est partiellement extériorisée par simple traction suivant l'orientation de la flèche 50 par le praticien. Cette orientation est généralement le prolongement hors des tissus du trajet de l'aiguille 17 en leur sein. L'aiguille 17 est ainsi tractée, de manière que l'extrémité opposée de l'aiguille ainsi que le fil 30 dans sa totalité (y compris le noeud 29) restent à tout moment à l'intérieur des tissus 49. Le repère 64, 65 visuel (encoche ou trait) proche de chaque extrémité de l'aiguille 17 permet le cas échéant de guider le praticien afin de lui éviter de trop extérioriser son aiguille. Une grande partie 51 de l'aiguille se situe alors à l'extérieur des tissus 49, tandis qu'une petite partie 52 de l'aiguille 17, à l'intérieur de laquelle se trouve le noeud 29, se situe toujours à l'intérieur des tissus 49. Le noeud 29 est à ce moment bloqué contre l'extrémité 37 de la fente 26.

Dans l'étape suivante représentée sur la figure 3b, le praticien imprime un mouvement de levier et de rotation sur la grande partie 51 de l'aiguille 17, de manière à faire pivoter l'ensemble de l'aiguille 17 suivant un angle 53. Cet angle 53 est déterminé par la trajectoire que le praticien souhaite imposer à l'aiguille 17. Comme les tissus 49 sont de manière générale souples et élastiques, le praticien n'a aucune difficulté pour orienter l'aiguille 17 dans la direction souhaitée.

Après la rotation, la plus grande partie 51 de l'aiguille se situe toujours à l'extérieur des tissus 49, tandis que la plus petite partie 52, à l'intérieur de laquelle se trouve le noeud 29, se situe toujours à l'intérieur des tissus 49. Le noeud 29 est toujours bloqué contre l'extrémité 37 de la fente 26.

Comme représenté sur la figure 3c, une fois que l'orientation de l'aiguille 17 correspond à celle souhaitée, le praticien peut ré-enfouir l'aiguille 17 dans les tissus suivant cette orientation schématisée par la flèche 54. Pour éviter de se blesser lorsqu'il pousse l'aiguille, le praticien peut recouvrir d'un capuchon 55 la pointe 23 qui se situe à l'extérieur des tissus 49. Ce capuchon 55 comporte généralement une forme complémentaire de celle des pointes 23 et 24, sans que son utilisation ne risque de les émousser. Dans un exemple, le capuchon 55 est réalisé en matière plastique.

Lorsque l'aiguille 17 s'enfonce à l'intérieur des tissus 49, elle n'entraîne pas immédiatement le fil 30. En effet, dans un premier temps, le noeud ou arrêtoir 29 coulisse alors le long de la fente 26 sans qu'il y ait de véritable déplacement du fil 30 au sein des tissus. Puis, lorsque le noeud 29 atteint puis se bloque contre l'extrémité 34 opposée à l'extrémité 37 de la fente 26, le fil 30 est de nouveau entraîné par l'aiguille 17. Ce fil 30 suit alors de nouveau la trajectoire imposée par l'aiguille 17.

Ainsi, comme représenté sur la figure 3d, l'aiguille 17 peut continuer de faire suivre au fil 30 la trajectoire souhaitée par le praticien. L'aiguille 17 peut suivre des trajectoires sinueuses, avec des rayons de courbures quelconques, sans que le fil 30 ne quitte les profondeurs des tissus 49. En outre, les ponctures provoquées par l'aiguille 17 lors de ses passages successifs de la barrière cutanée laissent très peu de traces après l'intervention, cicatrisant parfaitement en peu de temps, tout comme les suites de simple perfusions ou injections.

Les figures 4 montrent des représentations schématiques de variantes de réalisations de l'aiguille 17.

Sur la figure 4a, l'aiguille 17 comporte des pointes 23,24 biseautées de manière symétrique par rapport à un axe sagittal 58 et aux dépens de la face dorsale 22. Plus précisément, les angles 40 et 43 aigus des pointes 23 et 24 se situent du côté de la fente 26.

Sur la figure 4b, les pointes 23 et 24 sont biseautées de manière asymétrique par rapport à l'axe 58. Ainsi, l'angle aigu 40 de la pointe 23 se situe du côté de l'orifice 25, tandis que l'angle aigu 43 de la pointe 24 se situe du côté de la fente 26.

La figure 5a montre une représentation schématique d'un exemple d'ensemble fil/arrêtoir pouvant être utilisé avec l'aiguille 17 selon l'invention. Ce fil 59 comporte un arrêtoir 60 de diamètre 63 intermédiaire entre la plus petite dimension de l'orifice 25 et la plus grande dimension de la largeur de la fente 26. Plus généralement, lorsque l'arrêtoir 60 possède une forme quelconque, il possède des dimensions intermédiaires entre la plus petite dimension de l'orifice 25 et la plus grande dimension de la largeur de la fente 26.

Cet arrêtoir 60 peut être serti avec une extrémité 61 du fil 60. Plus précisément, un manchon métallique 62 relié à cet arrêtoir 60 est écrasé à l'aide d'un outil, tel qu'une pince, de manière à compresser l'extrémité 61 du fil 59 et le bloquer. L'arrêtoir 60 est réalisé dans un matériau, tel que l'acier, résistant à des contraintes de cisaillement.

La figure 5b représente un fil, spécialement conçu, présentant un renflement 63 à une extrémité formant arrêtoir. Ce renflement 63 peut par exemple être obtenu par moulage, en ajoutant un surplus de matière à une extrémité d'un moule d'un fil prévu à cet effet. Ce renflement 63 s'étend de manière radiale par rapport à un axe 66 du fil et peut par exemple avoir la forme d'une boule.

Bien sûr, les fils utilisés dans l'invention peuvent être de différentes natures, comme par exemple lisses ou crantés. Pour faciliter l'introduction dans la fente 26 de fils crantés, il est possible de les entourer d'une gaine lisse flexible. De cette manière, les crans du fil, qui s'étendent radialement par rapport à l'axe du fil et qui gène l'introduction du fil, n'entreront pas en contact avec un rebord de l'aiguille 17. L'utilisation de la gaine est particulièrement avantageuse avec une aiguille dépourvue d'orifice 25.

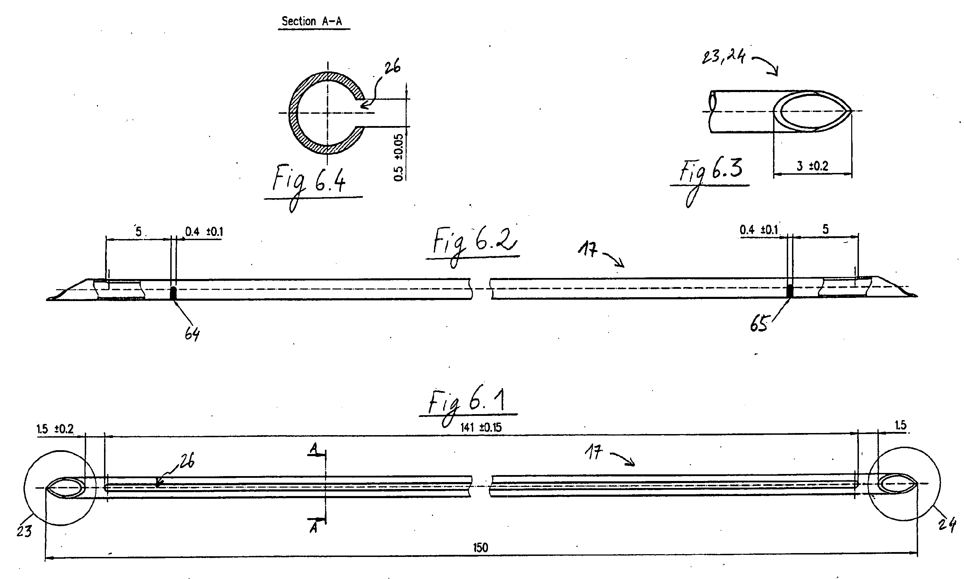

Les figures 6 montrent des représentations schématiques précises d'une aiguille 17 selon l'invention sans orifice. Les dimensions indiquées sur ces figures sont données en millimètres.

La figure 6.1 montre une vue de dessus de l'aiguille 17 représentée à l'échelle 5 :1. La figure 6.2 montre une vue de côté de l'aiguille 17. La figure 6.3 montre une vue en détail des pointes biseautées 23, 24 représentées à l'échelle 10 :1. La figure 6.4 montre une vue en coupe A-A de l'aiguille 17 à l'échelle 20 :1.

Plus précisément, dans cette réalisation, l'aiguille 17 mesure 150mm et est formée à partir d'un tube en acier inoxydable de diamètre de 1,2 à 1,5mm. Tandis que la fente 26, centrée par rapport à l'aiguille, possède une longueur de 141mm et une largeur de 0.5mm. Cette fente axiale 26 est orientée avec les biseaux à plus ou moins 5 degrés.

En outre, les pointes en biseau 23 et 24 sont de forme elliptique, et s'étendent sur une longueur de 3mm environ. Les pointes 23 et 24 comportent chacune un biseau à facettes selon UN-1050 et sont orientés à plus ou moins 5 degrés par rapport à une paroi de l'aiguille 17. Toutes ces valeurs sont données avec une tolérance de 10% environ.

Les encoches 64 et 65 sont réalisées au laser sur la moitié du contour de l'aiguille 17, soit 180 degrés, à plus ou moins 5 degrés. Ces encoches possèdent en générale la même orientation que les biseaux.

## Revendications

1. Aiguille chirurgicale (17) comportant :
- un corps creux (18) de forme allongée et deux extrémités (19, 20); ce corps (18) comportant une paroi (21) ventrale et une paroi dorsale (22) réunies entre elles, la paroi (22) dorsale étant opposée géométriquement à la paroi ventrale (21), cette parol dorsale (22) et cette paroi ventrale (21) étant allongées selon un allongement de l'aiguille (17), les deux extrémités (19, 20) creuses comportant chacune une pointe (23,24),
**caractérisée en ce qu'**elle comporte en outre :
- une fente (26) ménagée dans la paroi (21) ventrale, cette fente (26) s'étendant entre les deux extrémités (19, 20) du corps (18), cette fente (26) étant allongée dans le sens de l'allongement de aiguille (17), une longueur de cette fente (26) étant supérieure à 10% de la longueur de l'aiguille.

2. Aiguille selon la revendication 1, **caractérisée en ce que :**
- les pointes des extrémités sont creuses.

3. Aiguille selon la revendication 1 ou 2, **caractérisée en ce que :**
- une longueur de cette fente (26) est supérieure à 5 fois le diamètre de l'aiguille.

4. Aiguille selon l'une des revendications 1 à 3, **caractérisée en ce qu'**elle comporte en outre un orifice (25) ménagé dans la paroi (22) dorsale du corps (18).

5. Aiguille selon la revendication 4, **caractérisée en ce que :**
- la longueur de la fente (26) est supérieure à la plus grande dimension de l'orifice (25).

6. Aiguille selon la revendication 4 ou 5, **caractérisée en ce que :**
- une plus petite dimension (27) de l'orifice (25) est supérieure à une plus grande largeur (28) de la fente (26).

7. Aiguille selon l'une des revendications 4 à 6, **caractérisée en ce que :**
- l'orifice (25) est circulaire et possède un diamètre (27) supérieur à un diamètre des fils chirurgicaux usuels.

8. Aiguille selon l'une des revendications 4 à 7, **caractérisée en ce que :**
- l'orifice (25) possède des dimensions de l'ordre du millimètre, variables en fonction des dimensions de l'aiguille.

9. Aiguille selon l'une des revendications 1 à 8, **caractérisée en ce que :**
- la fente (26) est de forme globalement elliptique.

10. Aiguille selon l'une des revendications 1 à 9, **caractérisée en ce que:**
- ses extrémités (19,20) se terminent chacune par une pointe biseautée (23,24), de manière que la terminaison de celle-ci constitue le prolongement de l'une des deux parois (22,23), généralement de manière symétrique.

11. Aiguille selon l'une des revendications 1 à 10, **caractérisée en ce que :**
- le corps (18) comporte une forme cylindrique.

12. Aiguille selon l'une des revendications 1 à 11, **caractérisée en ce que :**
- le corps (18) comporte un diamètre (45) de l'ordre du millimètre et une longueur (46) comprise entre 5 et 20 cm.

13. Aiguille selon l'une des revendications 1 à 12, **caractérisée en ce que :**
- la fente (26) s'étend sur une longueur (47) inférieure de 5 à 15 mm à celle de la paroi dans laquelle elle se situe.

14. Aiguille selon l'une des revendications 1 à 13, **caractérisée en ce qu'**elle comporte un capuchon (55), ce capuchon (55) comportant une forme complémentaire de celle des pointes (23, 24) et étant destiné à recouvrir une de celles-ci pendant les manipulations.

15. Aiguille selon l'une des revendications 1 à 14, **caractérisée en ce que :**
- un chant (44) de la fente (26) qui se situe dans une épaisseur de l'aiguille (17) est arrondi ou recouvert d'un matériau non tranchant.

16. Aiguille selon l'une des revendications 1 à 15, **caractérisée en ce qu'**elle est réalisée en acier, ou dans un autre matériau adapté à son utilisation.

17. Aiguille selon l'une des revendications 1 à 16, **caractérisée en ce qu'**elle comporte un repère (64, 65) visuel tel qu'une encoche ou un trait, coloré ou non, ce repère (64, 65) étant inséré proche de chaque extrémité (19, 20).

18. Fil (59) avec aiguille chirurgicale selon l'une des revendications 1 à 17, **caractérisé en ce qu'**il comporte un arrêtoir (60) de dimensions (63) intermédiaires entre la plus petite dimension de l'orifice (25) et la plus grande dimension de la largeur de la fente (26).

19. Fil selon la revendication 18, **caractérisé en ce que :**
- l'arrêtoir (60) comporte un manchon (62) métallique qui est serti avec une extrémité (61) de ce fil.

20. Fil (64) selon la revendication 18 ou 19, **caractérisé en ce qu'**il comporte un renflement (65) de l'une de ses extrémités formant arrêtoir, ce renflement (65) étant réalisé par moulage.

## Claims

1. A surgical needle (17) comprising:
a long hollow body (18) and two ends (19, 20); this body (18) comprising a ventral wall (21) and a dorsal wall (22) connected to one another, the dorsal wall (22) being geometrically opposite the ventral wall (21), this dorsal wall (22) and this ventral wall (21) being in line with the line of the needle (17), the two hollow ends (19, 20) each comprising a point (23, 24),
**characterised in that** it furthermore comprises:
- a slit (26) provided in the ventral wall (21), this slit (26) extending between the two ends (19, 20) of the body (18), this slit (26) lying in the direction of the length of the needle (17), a length of this slit (26) being superior to 10% of the length of the needle.

2. A needle according to claim 1, **characterised in that:**
- the points of the ends are hollow.

3. A needle according to one of claims 1 to 2, **characterised in that:**
- a length of this slit (26) is superior to 5 times the diameter of the needle.

4. A needle according to one of the claims 1 to 3, **characterised in that** it furthermore comprises a hole (25) provided in the dorsal wall (22) of the body (18).

5. Needle according to the claim 4, **characterised in that:**
- the length of the slit (26) is greater than the largest dimension of the hole (25).

6. A needle according to the claim 4 or 5, **characterised in that:**
- a smallest dimension (27) of the hole (25) is greater than the largest width (28) of the slit (26).

7. A needle according to one of claim 4 to 6, **characterised in that:**
- the hole (25) is circular and has a diameter (27) greater than a diameter of ordinary surgical sutures.

8. A needle according to one of claims 4 to 7, **characterised in that:**
- the hole (25) has dimensions in the order of millimetres, variable according to the dimensions of the needle.

9. A needle according to one of claims 1 to 8, **characterised in that:**
- the slit (26) is of an overall elliptical shape.

10. A needle according to one of claims 1 to 9, **characterised in that:**
- its ends (19, 20) each finish in a beveled point (23, 24), in such a way that the endings of these constitute the extension of one of the two walls (22, 23), generally in a symmetrical manner.

11. A needle according to one of claims 1 to 10, **characterised in that:**
- the body (18) comprises a cylindrical shape.

12. A needle according to one of claims 1 to 11, **characterised in that:**
- the body (18) comprises a diameter (45) in the order of millimetres and a length (46) included between 5 and 20 cm.

13. A needle according to one of claims 1 to 12, **characterised in that:**
- The slit (26) extends for example over a length (47) of less than 5 to 15mm to that of the wall in which it is situated.

14. A needle according to one of the claims 1 to 13, **characterised in that** it includes a cap (55), this cap (55) comprising a shape compatible with the points (23, 24) and being designed to cover one of these during manipulations.

15. A needle according to one of claims 1 to 14, **characterised in that:**
- an edge (44) of the slit (26) which is situated in a thickness of the needle (17) is rounded off or coated in a non-cutting material.

16. A needle according to one of the claims 1 to 15, **characterised in that** it is made of steel, or of another material adapted to its use.

17. A needle according to one the claims 1 to 16, **characterised in that** it comprises a visual marker (64, 65) such as a notch or a line, coloured or not, this marker (64, 65) being inserted close to each end (19, 20).

18. A suture (59) with the surgical needle according to one of the claims 1 to 17, **characterised in that** it comprises a suture stop (60) of dimensions (63)which are intermediary between the smallest dimension of the hole (25) and the greatest width of the slit (26).

19. A suture according to claim 18, **characterised in that:**
- the suture stop (60) includes a metallic sleeve (62) which is set with one end (61) of this suture.

20. A suture (64) according to one of the claims 19 to 20, **characterised in that** it includes a bulge (65) in one of its ends which serves as a suture stop, this bulge (65) being obtained by casting.

## Patentansprüche

1. Chirurgische Nadel (17) umfassend:
- einen hohlen Körper (18) von länglicher Form mit zwei Enden (19, 20), der eine ventrale Wand und eine dorsale Wand (22) umfasst, die miteinander verbunden sind, und wobei die dorsale Wand (22) geometrisch gegenüber der ventralen Wand (21) angeordnet ist und die dorsale Wand (22) und die ventrale Wand (21) länglich gemäß der Ausdehnung der Nadel (17) verlaufen und die beiden hohlen Enden (19, 20) jeweils eine Spitze (23, 24) aufweisen,
**dadurch gekennzeichnet, dass** sie zusätzlich aufweist:
- einen Schlitz (26) in der ventralen Wand (21), der sich zwischen den beiden Enden (19, 20) des Körpers (18) länglich in Richtung der Ausdehnung der Nadel (17) erstreckt, und wobei eine Länge dieses Schlitzes (26) größer ist als 10% der Länge der Nadel.

2. Nadel nach Anspruch 1, **dadurch gekennzeichnet, dass:**
- die Spitzen der beiden Enden hohl sind.

3. Nadel nach Anspruch 1 oder 2, **dadurch gekennzeichnet, dass:**
- eine Länge des Schlitzes (26) größer als der fünffache Durchmesser der Nadel ist.

4. Nadel nach einem der Ansprüche 1 bis 3, **dadurch gekennzeichnet, dass:**
- sie außerdem eine Öffnung (25) in der dorsalen Wand (22) des Körpers (18) aufweist.

5. Nadel nach Anspruch 4, **dadurch gekennzeichnet, dass:**
- die Länge des Schlitzes (26) größer ist als die größte Dimension der Öffnung (25).

6. Nadel nach Anspruch 4 oder 5, **dadurch gekennzeichnet, dass:**
- eine kleinste Dimension (27) der Öffnung (25) größer als eine größere Dimension (28) des Schlitzes (26) ist.

7. Nadel nach einem der Ansprüche 4 bis 6, **dadurch gekennzeichnet, dass:**
- die Öffnung (25) kreisförmig ist und einen Durchmesser (27) aufweist, der größer ist als ein Durchmesser eines üblichen chirurgischen Fadens.

8. Nadel nach einem der Ansprüche 4 bis 7, **dadurch gekennzeichnet, dass:**
- die Öffnung (25) Dimensionen von der Größenordnung eines Millimeters aufweist, abhängig von der Dimension der Nadel.

9. Nadel nach einem der Ansprüche 1 bis 8, **dadurch gekennzeichnet, dass:**
- der Schlitz (26) von insgesamt elliptischer Form ist.

10. Nadel nach einem der Ansprüche 1 bis 9, **dadurch gekennzeichnet, dass:**
- ihre Enden (19, 20) jeweils mit einer abgeschrägten Spitze (23, 24) enden, so dass die Endung eine Verlängerung von einer der beiden Wände (22, 23) in im Wesentlichen symmetrischer Art darstellen.

11. Nadel nach einem der Ansprüche 1 bis 10, **dadurch gekennzeichnet, dass:**
- der Körper (18) eine zylindrische Form aufweist.

12. Nadel nach einem der Ansprüche 1 bis 11, **dadurch gekennzeichnet, dass:**
- der Körper (18) einen Durchmesser (45) von der Größenordnung eines Millimeters und eine Länge (46) zwischen 5 und 20 cm aufweist.

13. Nadel nach einem der Ansprüche 1 bis 12, **dadurch gekennzeichnet, dass:**
- der Schlitz (26) sich über eine Länge (47) erstreckt, die 5 bis 15 mm geringer ist als die der Wand, in der er sich befindet.

14. Nadel nach einem der Ansprüche 1 bis 13, **dadurch gekennzeichnet, dass:**
- sie eine Kappe (55) aufweist, die eine Form komplementär zu der der Spitzen (23, 24) aufweist und dazu bestimmt ist, eine davon während der Handhabung abzudecken.

15. Nadel nach einem der Ansprüche 1 bis 14, **dadurch gekennzeichnet, dass:**
- eine Kante (44) des Schlitzes (26), der sich in einer Dicke der Nadel (17) befindet, abgerundet oder mit einem nicht schneidenden Material bedeckt ist.

16. Nadel nach einem der Ansprüche 1 bis 15, **dadurch gekennzeichnet, dass:**
- sie aus Stahl oder einem anderen an ihren Verwendungszweck angepassten Material besteht.

17. Nadel nach einem der Ansprüche 1 bis 16, **dadurch gekennzeichnet, dass:**
- sie eine sichtbare Markierung (64, 65) aufweist, wie eine Kerbe oder einen farbigen oder nicht-farbigen Strich, der in der Nähe jedes Endes (19, 20) angeordnet ist.

18. Nahtmaterial mit chirurgischer Nadel gemäß einem der Ansprüche 1 bis 17, **dadurch gekennzeichnet, dass** es eine Arretierung (60) aufweist von einer Dimension (63) zwischen der kleinsten Dimension der Öffnung (25) und der größten Dimension der Länge des Schlitzes (26).

19. Nahtmaterial nach Anspruch 18, **dadurch gekennzeichnet, dass** die Arretierung (60) einen metallischen Ärmel (62) aufweist, der ein Ende (61) des Nahtmaterials einfasst.

20. Nahtmaterial nach Anspruch 18 oder 19, **dadurch gekennzeichnet, dass** es eine Verdickung (65) eines seiner Enden aufweist, die eine Arretierung bildet, wobei die Verdickung (65) durch Formguss hergestellt ist.
